# EUROPÄISCHE PATENTSCHRIFT

(11) **EP 2 475 466 B1**
(45) Veröffentlichungstag und Bekanntmachung des Hinweises auf die Patenterteilung: **09.11.2016**
(21) Anmeldenummer: 10754871.1
(22) Anmeldetag: 09.09.2010
(51) Int. Cl.: B05B 11/00, B65D 83/00, B65D 51/28, A61M 5/14, A61M 5/28, A61M 5/315, B05B 11/02, A61M 5/31, A61M 5/34

(54) **DOSIERVORRICHTUNG ZUR DOSIERTEN AUSGABE VON FLÜSSIGPRÄPARATEN, VERFAHREN ZUR BEFÜLLUNG SOWIE VERWENDUNG EINER ERFINDUNGSGEMÄSSEN DOSIERVORRICHTUNG**
METERING DEVICE FOR THE METERED DISPENSION OF FLUID PREPARATIONS, METHOD FOR FILLING AND USE OF A METERING DEVICE ACCORDING TO THE INVENTION
DISPOSITIF DE DOSAGE POUR LA DISTRIBUTION DOSÉE DE PRÉPARATIONS LIQUIDES, PROCÉDÉ DE REMPLISSAGE AINSI QU'UTILISATION D'UN DISPOSITIF DE DOSAGE SELON L'INVENTION

(30) Priorität: 09.09.2009 DE 102009040783
(43) Veröffentlichungstag der Anmeldung: 18.07.2012
(73) Patentinhaber: KIST-Europe Forschungsgesellschaft mbH, 66123 Saarbrücken (DE)
(72) Erfinder: LEE, Hyeck, Hee, 66386 St. Ingbert (DE); STEINFELD, Ute, 66386 St. Ingbert (DE); KIM, Jungtae, 66121 Saarbrücken (DE); KRAUSE, Holger, 66540 Neunkirchen (DE); PARK, Jihwang, Seoul 120-070 (KR); KIM, Kwang Ho, 66123 Saarbrücken (DE); SCHWEIGER, Bianca, 66111 Saarbrücken (DE); BECK, Sebastian, 66386 St. Ingbert (DE)
(74) Vertreter: Pfenning, Meinig & Partner mbB
(86) Internationale Anmeldenummer: PCT/EP2010/005557
(87) Internationale Veröffentlichungsnummer: WO 2011/029604

(56) Entgegenhaltungen:
- DE-U1- 29 717 034
- IL-A- 46 017
- US-A- 3 655 096
- US-A1- 2006 052 747
- US-A1- 2008 011 779

## Beschreibung

Die vorliegende Erfindung betrifft eine Dosiervorrichtung zur dosierten Ausgabe von Flüssigpräparaten im medizinischen, pharmazeutischen oder kosmetischen Bereich oder im Lebensmittelbereich, wobei auf die Verwendung von Konservierungsmitteln verzichtet werden kann. Des Weiteren betrifft die Erfindung ein Verfahren zur Befüllung sowie die Verwendung einer erfindungsgemäßen Dosiervorrichtung.

Die aus dem Stand der Technik bekannten Dosiervorrichtungen enthalten ein Vorratsbehältnis sowie eine Dosierpumpe. Ein Beispiel für eine Dosierpumpe wird in der DE 10 2008 027 987 gegeben. Die dort beschriebene Dosierpumpe enthält einen mit einer Kappe verbundenen Hohlkörper, einen Pumpkolben, eine Pumpkammer sowie eine mit der Pumpkammer über einen Auslasskanal in Verbindung stehende Düse. In Kombination mit einer solchen Dosierpumpe werden Vorratsbehältnisse mit Druckausgleich verwendet.

Die aus dem Stand der Technik bekannten Dosiervorrichtungen sind häufig mit geeigneten Dichtungen versehen, so dass der Inhalt des Vorratsbehältnisses hermetisch abgedichtet ist. Dennoch ist der Einsatz von Konservierungsstoffen bei der Lagerung einer Vielzahl von flüssigen Präparaten bisher unumgänglich. Der Einsatz von Konservierungsstoffen führt jedoch zu Unverträglichkeiten bei den Verbrauchern, insbesondere wenn diese Allergiker sind.

Da die einzelnen Inhaltsstoffe eines Präparates bei getrennter Lagerung oftmals länger haltbar sind, werden durch den Stand der Technik Sets vorgeschlagen, die dem Verbraucher vor der ersten Benutzung ein eigenhändiges Vermischen der einzelnen Inhaltsstoffe ermöglicht. Solche Sets sind jedoch schwierig handhabbar und beim Mischen der Inhaltsstoffe kann es zu Kontaminationen des endgültigen Präparats kommen. Eine Dosiervorrichtung gemäß den Oberbegriff des Anspruchs 1 ist aus 16 46017 bekannt.

Ausgehend vom Stand der Technik ist es nun die Aufgabe der vorliegenden Erfindung, eine Dosiervorrichtung sowie ein Verfahren zur Befüllung und eine Verwendung einer Dosiervorrichtung zur Verfügung zu stellen, welche eine verlängerte Haltbarkeit eines Präparates ermöglichen. Außerdem soll die Dosiervorrichtung einfach und günstig herstellbar sein und insbesondere für den Verbraucher einfach handzuhaben.

Diese Aufgaben werden durch die Dosiervorrichtung nach Anspruch 1 sowie deren Verfahren zum Betreiben nach Anspruch 14 und deren Verwendung nach Anspruch 16 gelöst. Vorteilhafte Weiterbildungen der vorliegenden Erfindung werden in den abhängigen Ansprüchen beschrieben.

Erfindungsgemäß weist die Dosiervorrichtung zur dosierten Ausgabe eines flüssigen Präparates, welches mindestens zwei Ausgangssubstanzen enthält, ein Vorratsgefäß zur Aufnahme zumindest der ersten Ausgangssubstanz mit einer Öffnung, ein Innengefäß zur Aufnahme der zweiten Ausgangssubstanz sowie eine Ausgabeeinheit mit einem Durchlasskanal mit zwei Kanalöffnungen auf. Das Innengefäß ist im Bereich der Öffnung des Vorratsgefäßes so angeordnet, dass es das Vorratsgefäß abdichtet. Die Ausgabeeinheit ist mit einer ihrer Kanalöffnungen, welche im Folgenden als Einlassöffnung bezeichnet wird, im Bereich der Vorratsgefäßöffnung dem Vorratsgefäß zugewandt angeordnet und dichtend mit dem Vorratsgefäß verbunden. Weiterhin ist der dem Vorratsgefäß zugewandte Bereich der Ausgabeeinheit als Durchstoßeinheit ausgebildet, welche in die Öffnung des Vorratsgefäßes einführbar und/oder in dieser führbar ist. Im Bereich der Öffnung des Vorratsgefäßes zwischen Vorratsgefäß und Ausgabeeinheit ist ein, zumindest gegenüber dem Vorratsgefäß, abgedichtetes Innengefäß zur Aufnahme der zweiten Substanz angeordnet. Durch Einführung und/oder Führung der Durchstoßeinheit in die und/oder in der Öffnung ist das Innengefäß mit Hilfe der Durchstoßeinheit durchstoßbar und die Dosiervorrichtung damit aktivierbar.

Dabei ist sowohl vor der ersten Benutzung als auch während der Benutzung eine dichtende Verbindung zwischen der Ausgabeeinheit, dem Vorratsgefäß sowie dem Innengefäß gewährleistet. Diese ist, unabhängig von den Betriebszuständen sowie von den Umgebungsbedingungen, gegeben. Dadurch kann beispielsweise eine Kontamination des Inhalts effektiv vermieden werden.

Vor der ersten Benutzung der erfindungsgemäßen Dosiervorrichtung befindet sich die Dosiervorrichtung in einer nicht aktivierten Position, d.h. die Durchstoßeinheit ist beabstandet von dem Innengefäß angeordnet und das Innengefäß ist noch nicht durchstoßen, so dass die zweite Ausgangssubstanz noch von der ersten Ausgangssubstanz getrennt gelagert wird.

Die Dosiervorrichtung weist insbesondere vor als auch während der Aktivierung der Dosiervorrichtung eine dichtende Verbindung zwischen dem Vorratsgefäß und der Ausgabeeinheit auf. Somit kann zu keinem Zeitpunkt Umgebungsluft an den Inhalt der Dosiervorrichtung gelangen.

Durch die Trennung der Substanzen vor dem Gebrauch kann während der Lagerung auf Konservierungsmittel verzichtet werden.

Durch Aktivieren der Dosiervorrichtung gelangt diese in eine Gebrauchsposition, in welcher das Innengefäß durchstoßen ist und der Inhalt des Innengefäßes in das Vorratsgefäß eingebracht ist. Die Gebrauchsposition kann beispielsweise durch einen Farbstoff angezeigt werden, wobei sich als Farbstoff insbesondere allgemein verträgliche Naturfarbstoffe anbieten.

Die Idee der vorliegenden Erfindung beruht also auf dem Prinzip, die Ausgangssubstanzen bis zu einem ersten Gebrauch getrennt voneinander aufzubewahren und bevorzugt jeweils hermetisch abzudichten. Die Haltbarkeit des Inhalts der Dosiervorrichtung wird also verlängert, indem das endgültige flüssige Präparat durch Mischung der ersten und der zweiten Ausgangssubstanz erst beim ersten Gebrauch hergestellt wird.

Der Inhalt des Innengefäßes sowie des Vorratsgefäßes sind vorzugsweise ab dem Hersteller, während und nach der Aktivierung hermetisch abgedichtet. Die hermetische Versiegelung des Vorratsgefäßes kann durch Anbringen, insbesondere durch Aufschweißen oder Aufkleben, des Innengefäßes in der Vorratsgefäßöffnung erfolgen. Der Übergang zwischen Vorratsgefäß und Ausgabeeinheit ist vorzugsweise mit einer Dichtung abgedichtet und der Kanal der Ausgabeeinheit weist zur hermetischen Abdichtung des Inneren der Ausgabeeinheit bevorzugt an der dem Vorratsgefäß abgewandten Kanalöffnung eine Düse oder eine zusätzliche Verschlusskappe mit Dichtwirkung auf.

Vorzugsweise sind die Ausgangssubstanzen so gewählt, dass die erste Ausgangssubstanz eine flüssige Substanz, bevorzugt Wasser, ist, während die zweite Ausgangssubstanz ein Pulver oder eine ölige emulgierbare Substanz ist. Vorzugsweise sind die erste und zweite Substanz so gewählt, dass die zweite Substanz in der ersten Substanz lösbar oder emulgierbar ist oder dass die zweite Substanz mit der ersten Substanz reagiert.

Die Aktivierung, d.h. der Übergang von der nicht aktivierten Position in die Gebrauchsposition, ist durch eine Bewegung der Ausgabeeinheit in Richtung des Vorratsgefäßes und in Richtung des Innengefäßes erreichbar. Eine solche Bewegung ist insbesondere durch Drücken oder Drehen realisierbar.

Die Öffnung des Vorratsgefäßes kann kreisförmig, oval oder mehreckig ausgebildet sein. Das Vorratsgefäß kann beispielsweise als Behältnis mit entsprechendem Querschnittsprofil wie die Öffnung ausgebildet sein. Alternativ kann das Behältnis aber auch einen sich von der Öffnung unterscheidenden Querschnitt aufweist, beispielsweise eine eckige Öffnung und ein Behältnis mit kreisförmigem Querschnitt.

Vorzugsweise ist die Öffnung des Vorratsbehältnisses als Hals ausgebildet. In einer solchen Ausführungsform kann das Innengefäß in einem dem Vorratsgefäß zugewandten Bereich des Halses angeordnet sein, während die Durchstoßeinheit der Ausgabeeinheit im nicht aktivierten Zustand der Dosiervorrichtung zumindest teilweise im vom Vorratsgefäß abgewandten Bereich des Halses angeordnet sein kann.

Die Dosiervorrichtung ist so ausgebildet, dass die Ausgabeeinheit nach dem Bewegen dieser in Richtung des Vorratsgefäßes und nach dem Durchstoßen des Innengefäßes mit dem Vorratsgefäß verrastet ist. Dazu weist die Ausgabeeinheit vorzugsweise einen Hohlzylinder oder ein Hohlprisma auf, welcher oder welches die Durchstoßeinheit umgibt. Der Hohlzylinder ist dabei bevorzugt als entlang seiner gesamten Länge ausgehöhlter Zylinder mit geeigneter Grundfläche, beispielsweise mit kreisförmiger, ovaler oder bananenförmiger Grundfläche, ausgebildet. Der Hohlzylinder bzw. das Hohlprisma weisen an ihrer Innenseite zumindest bereichsweise Hinterschneidungen auf. Außerdem weist das Vorratsgefäß bevorzugt im Bereich seiner Öffnung, insbesondere im Falle einer als Hals geformten Öffnung, Hinterschneidungen auf. Werden nun Ausgabeeinheit und Vorratsgefäß aufeinander zu bewegt, so greifen die Hinterschneidungen des Hohlzylinders bzw. des Hohlprismas und des Vorratsgefäßes ineinander oder jeweils mit einer geeignet geformten Dichtung ein und die Ausgabeeinheit rastet auf das Vorratsgefäß auf.

Zur wasser- und luftdichten Verbindung der Ausgabeeinheit mit dem Vorratsgefäß weist die Dosiervorrichtung einen flexiblen Kunststoffschlauch als Dichtung auf, wobei der Kunststoffschlauch unter anderem die dichte Verbindung von Vorratsgefäß und Ausgabeeinheit herstellen kann. Das eine Ende des Kunststoffschlauches umgibt die Öffnung des Vorratsgefäßes und das zweite Ende des Schlauches ist so angeordnet, dass es insbesondere die Einlassöffnung des Kanals der Ausgabeeinheit umgibt. Dabei ist das der Ausgabeeinheit zugewandte Ende des Schlauches bevorzugt mit dem die Durchstoßeinheit umgebenden Hohlzylinder oder Hohlprisma in Verbindung. Falls das Vorratsgefäß aus Kunststoff gefertigt ist, kann dieses einteilig mit dem Kunststoffschlauch ausgebildete sein.

Der Kunststoffschlauch weist einen oder zwei Bereiche mit jeweils mindestens einer Verdickung auf. Die Verdickung zieht sich bevorzugt über den gesamten Umfang des jeweiligen Schlauchbereiches entlang. Die jeweiligen Verdickungen greifen in die Hinterschneidungen des Hohlzylinders bzw. des Hohlprismas und/oder des Vorratsgefäßes ein. Im aktivierten Zustand ist der flexible Kunststoffschlauch nach innen in die Ausgabeeinheit, insbesondere in einen Bereich zwischen Durchstoßeinheit und Hohlzylinder oder Hohlprisma der Ausgabeeinheit eingerollt, so dass die Verdickung in dem, dem Vorratsgefäß zugewandten Bereich und/oder die Verdickung in dem, der Ausgabeeinheit zugewandten Bereich ineinander eingreifen und die Ausgabeeinheit auf dem Vorratsgefäß aufrastet. Damit wird beispielsweise eine verbesserte Dichtigkeit zwischen dem Vorratsgefäß und der Ausgabeeinheit hergestellt.

Die Durchstoßeinheit kann als angespitzter Einlassstutzen, dessen eines Ende in die Einlassöffnung der Ausgabeeinheit mündet, ausgebildet sein. Zusätzlich kann der Einlassstutzen an seinem der Einlassöffnung abgewandten Ende einen Schneidekranz, einen Sägekranz, einen Zackenkranz oder zumindest eine zum Durchstoßen des Innengefäßes geeignete Zinke aufweisen. Alternativ kann die Durchstoßeinrichtung auch als separater Schneidekranz, Sägekranz, Zackenkranz, insbesondere als schräger Schneidekranz, Sägekranz oder Zackenkranz, oder zumindest eine zum Durchstoßen des Innengefäßes geeignete Zinke oder als Dorn, welcher bevorzugt in der Nähe der Einlassöffnung der Ausgabeeinheit angeordnet ist, ausgebildet sein.

Die erfindungsgemäße Dosiervorrichtung kann zum Aussprühen einer flüssigen Substanz verwendet werden. Dazu ist die Ausgabeeinheit vorzugsweise als Dosierpumpe, wie sie aus dem Stand der Technik bekannt ist, ausgebildet.

Vorzugsweise enthält eine in Verbindung mit der erfindungsgemäßen Dosiervorrichtung verwendete Dosierpumpe einen Einlasskanal, einen Pumpkolben, eine Pumpkammer und eine über einen Auslasskanal mit der Pumpkammer verbundene Düse, wobei der Einlasskanal und der Auslasskanal der Dosierpumpe in diesem Fall dem Kanal der Ausgabeeinheit entsprechen. Durch die Einlassöffnung kann das flüssige Präparat aus dem Vorratsgefäß in die Pumpkammer eingeleitet werden. Durch Bewegen des Pumpkolbens wird die Flüssigkeit aus der Pumpkammer durch den Auslasskanal und die Düse nach außen befördert.

Je nach Anwendungsgebiet kann die Düse im Pumpkopf so angeordnet sein, dass die Flüssigkeit an der vom Vorratsgefäß abgewandten Seite oder seitlich ausgegeben wird.

Alternativ kann das Sprühen der Flüssigkeit aus dem Vorratsgefäß auch durch Verpressen eines elastischen Vorratsgefäßes erfolgen. Die Ausgabeeinheit ist dann vorzugsweise als Ausgabekappe mit Kanal, in welchem eine Düse angeordnet ist, ausgebildet. Nach dem Durchstoßen des Innengefäßes kann durch Eindrücken der Wände des Vorratsgefäßrumpfes die Flüssigkeit aus dem Vorratsgefäß gesprüht werden.

Außerdem kann die erfindungsgemäße Dosiervorrichtung auch als Einwegspritze ausgebildet sein. Die Ausgabeeinheit ist in diesem Fall vorzugsweise lediglich als Ausgabekappe, welche einen Kanal aufweist, ausgebildet. Die Einwegspritze weist einen Presskolben auf, welcher den von der Ausgabekappe abgewandten Boden des Vorratsgefäßes bildet. Nach dem Durchstoßen des Innengefäßes kann eine Kanüle auf die Ausgabekappe aufgesteckt werden. Durch Bewegen des Presskolbens in Richtung der Ausgabekappe kann die Flüssigkeit durch den Kanal der Ausgabekappe aus dem Vorratsgefäß gespritzt werden.

Unabhängig von der Ausformung der Ausgabeeinheit ist das Vorratsgefäß vorzugsweise als Behältnis ausgebildet, welches eine Druckausgleichsvorrichtung aufweist. Beispielsweise kann ein Beutel, ein Faltenbalg oder eine Flasche mit Druckausgleich, insbesondere eine Schleppkolbenflasche, oder eine Spritze, wobei der Presskolben als Druckausgleichsvorrichtung dient, eingesetzt werden.

Vorzugsweise enthält das Vorratsgefäß eine Flasche mit einer Druckausgleichsöffnung, in welcher ein Faltenbalg mit Boden eingesetzt ist. Alternativ kann in der Flasche mit Druckausgleichsöffnung ein Schleppkolbenboden vorliegen, welcher bevorzugt mittels eines bodenlosen Faltenbalgs und/oder einer Feder bewegbar ist.

Als Materialien für das Vorratsgefäß werden insbesondere Materialien gewählt, welche keine Reaktion mit der ersten Ausgangssubstanz sowie mit dem flüssigen Präparat zeigen. Insbesondere kommen Glas- oder Kunststoffbehältnisse zum Einsatz.

Das Innengefäß ist vorzugsweise so gestaltet, dass es chemisch beständig ist, eine geringe Feuchtigkeitsabsorption aufweist und/oder in einem weiten Temperaturbereich verwendbar ist. Das Innengefäß besteht daher bevorzugt aus einem Metall, insbesondere Aluminium, aus einem Kunststoff oder aus einem Glas oder enthält eines dieser Materialien. Als Beispiel für einen Kunststoff kann beispielsweise Polychlortrifluorethylen (PCTFE)genannt werden, welches nicht entflammbar ist, chemisch beständig ist, eine Feuchtigkeitsabsorption nahe Null aufweist und bei Temperaturen in einem Bereich von -240°C bis 209°C verwendbar ist. Neben PCTFE sind auch PE/PCTFE/PE-Mehrschicht-Folien möglich (PE = Polyethylen). Auch eine einseitig laminierte PE/PCTFE-Folie ist denkbar. Alternativ bieten sich Cyclo-Olefin-Copolymere (COC) an, welche zwar schlechtere Barriereeigenschaften als PCTFE aufweisen, dagegen aber preiswerter sind. COC sind außerdem kratzempfindlich und können daher als Mehrschicht-Folie beidseitig mit PP (Polypropylen) beschichtet sein. Dabei dient PP sowohl als Schutzschicht für das kratzempfindliche COC und außerdem als Haftschicht. Weiterhin kann Aluminium mit Kunststoffbeschichtung (Schutz gegen Oxidieren) bzw. geeignete Polymerfolien oder auch Mehrschichtfolien eingesetzt werden.

Das Innengefäß ist als geschlossenes Gefäß ausgebildet, dessen Inhalt hermetisch abgedichtet ist. Das Innengefäß verschließt weiter den Inhalt des Vorratsgefäßes.

Das Innengefäß kann eine erste und eine zweite Schicht aufweisen, zwischen welchen der Inhalt des Innengefäßes abgedichtet ist. Die beiden Schichten sind an ihren Rändern dauerhaft miteinander und mit dem Vorratsgefäß verbunden, beispielsweise miteinander, und auf die Öffnung des Vorratsgefäßes aufgeschweißt oder aufgeklebt oder auflaminiert.

Sind die erste und zweite Schicht aus PCTFE hergestellt, so weisen sie eine Dicke im Bereich von einigen 10 µm bis einige 100 µm, insbesondere eine Dicke von 102 µm, auf.

Das Vorratsgefäß und/oder das Innengefäß und/oder der Kanal der Ausgabeeinheit können insbesondere in und/oder an ihren Innenwänden ein bakterizid wirkendes Mittel, insbesondere eine bakterizid wirkende Beschichtung oder bakterizid wirkende Einbauten, aufweisen. Bakterizid wirkende Mittel enthalten bevorzugt Silber oder Silbersalze, wie beispielsweise Silberchlorid.

Die Ausgabeeinheit kann im Bereich des Einlasskanals einen Filter aufweisen, welcher das endgültige Präparat von im endgültigen flüssigen Präparat enthaltenen Überresten des Innengefäßes reinigt und den Kanal der Ausgabeeinheit vor einem Verstopfen durch Überreste des Innengefäßes schützt.

Die Dosiervorrichtung kann gegebenenfalls zumindest ein Zusatzgefäß zur Aufnahme einer dritten Ausgangssubstanz oder zur Aufnahme eines Farbstoffes aufweisen. Ein solches Zusatzgefäß ist vorzugsweise im Bereich der Öffnung des Vorratsgefäßes auf der dem Vorratsgefäß zugewandten Seite des Innengefäßes oder auf der der Ausgabeeinheit zugewandten Seite des Innengefäßes oder neben dem Innengefäß angeordnet und mit Hilfe der Durchstoßeinheit der Ausgabeeinheit durchstoßbar. Besteht das endgültige flüssige Präparat also beispielsweise aus drei Ausgangssubstanzen, welche getrennt voneinander länger haltbar sind als in einem Gemisch, so sind in der Öffnung des Vorratsbehältnisses beispielsweise sowohl das Innengefäß als auch das Zusatzgefäß angeordnet. Bei der Aktivierung der Dosiervorrichtung werden das Innengefäß und das Zusatzgefäß durchstoßen, so dass deren Inhalt in das Vorratsgefäß eindringt und sich die drei Ausgangssubstanzen zu dem Präparat vermischen, wobei die Vermischung durch Schütteln beschleunigt werden kann.

Die Aktivierung der Ausgabeeinheit kann z.B. durch eine Drück- oder Schraubbewegung erfolgen.

Im Falle einer Aktivierung durch Drücken weist das Vorratsgefäß bevorzugt einen Hohlzylinder oder ein Hohlprisma auf, welche die Öffnung des Vorratsgefäßes umlaufen. Der Hohlzylinder ist dabei wieder bevorzugt als entlang seiner gesamten Länge ausgehöhlter Zylinder mit geeigneter Grundfläche, beispielsweise mit kreisförmiger, ovaler oder bananenförmiger Grundfläche, ausgebildet. Die Grundfläche des Hohlzylinders bzw. des Hohlprismas ist dabei vorzugsweise so gewählt, dass zumindest die Durchstoßeinheit und bevorzugt die Durchstoßeinheit und ein diese umlaufender Hohlzylinder oder Hohlprisma der Ausgabeeinheit in das Innere des Hohlzylinders bzw. des Hohlprismas des Vorrätsgefäßes einführbar ist. Der Hohlzylinder bzw. das Hohlprisma können, insbesondere während der Drückbewegung, als Führung für die Ausgabeeinheit dienen, um ein Verkanten zu vermeiden.

Soll die Aktivierung durch eine Schraubbewegung erfolgen, so weist die Dosiervorrichtung eine Schraubhülse zur Führung der Ausgabeeinheit in Richtung des Vorratsgefäßes auf. Eine solche Schraubhülse weist vorzugsweise ein oder mehrere Gewindegänge auf. Durch Drehen der Schraubhülse wird die Ausgabeeinheit in Richtung des Vorratsgefäßes gezogen.

Um die nicht aktivierte Position der Dosiervorrichtung vor einer unbeabsichtigten Aktivierung zu schützen, weist die Dosiervorrichtung vorzugsweise einen Sperrmechanismus auf, welcher beispielsweise als Sperrring zwischen dem Vorratsgefäß und der Ausgabeeinheit ausgebildet ist.

Zum Schutz vor Verschmutzung kann die Ausgabeeinheit zusätzlich mit einer Verschlusskappe versehen sein, die nach Aktivierung bevorzugt vor jeder Benutzung abgenommen und gegebenenfalls nach jeder Benutzung wieder aufgesetzt werden kann.

In der erfindungsgemäßen Dosiervorrichtung kann das Innengefäß und/oder das Vorratsgefäß Schutzgas und/oder Inertgas enthalten um den Inhalt vor Oxidation und/oder Hydrolyse zu schützen. Dies kann auch für ein gegebenenfalls vorliegendes Zusatzgefäß zutreffen.

Weiterhin kann die erste Ausgangssubstanz mit Schutzgas und/oder Inertgas, gegebenenfalls vor dem Befüllen, durchsetzt sein. Dies kann auch für eine zweite bzw. dritte Ausgangssubstanz gelten.

Das Innengefäß und/oder das Vorratsgefäß können bevorzugt unter einer Atmosphäre aus Schutzgas und/oder Inertgas befüllt sein. Auch ein gegebenenfalls vorliegendes Zusatzgefäß kann auf diese Weise befüllt sein.

Das Schutzgas kann hierbei ausgewählt sein aus Stickstoff N₂, Kohlenstoffdioxid CO₂ oder Mischungen hiervon.

Das Inertgas ist vorzugsweise ausgewählt aus Stickstoff, Edelgasen, wie z.B. Helium He oder Argon Ar, oder Mischungen hiervon.

Im Vorratsgefäß der erfindungsgemäßen Dosiervorrichtung kann einen Faltenbalg angeordnet sein, wodurch eine hermetische Abdichtung der Dosiervorrichtung bis zum Ende der Verwendung gewährleistet ist.

Die vorliegende Erfindung betrifft weiterhin ein Verfahren zur Befüllung einer erfindungsgemäßen Dosiervorrichtung. Dabei wird in einem ersten Schritt das Vorratsgefäß mit einer ersten Ausgangssubstanz befüllt. In einem zweiten Schritt wird das Innengefäß dauerhaft im Bereich der Öffnung des Vorratsgefäßes angebracht. Das Innengefäß kann dabei bereits vor dem Aufbringen gefüllt sein oder, insbesondere wenn das Innengefäß aus einer ersten und einer zweiten Schicht besteht, zwischen dem Aufbringen der ersten Schicht auf das Vorratsgefäß und dem Aufbringen der zweiten Schicht befüllt werden. In einem dritten Schritt wird abschließend die Ausgabeeinheit auf das Vorratsgefäß aufgesetzt. Enthält die Dosiervorrichtung einen flexiblen Kunststoffschlauch, so wird der Schlauch vor dem Aufsetzen der Ausgabeeinheit an dieser abdichtend befestigt und anschließend beim Aufsetzen der Ausgabeeinheit über die Öffnung des Vorratsgefäßes gesteckt.

Die Aktivierung der Ausgabeeinheit erfolgt durch Bewegen der Ausgabeeinheit Richtung des Vorratsgefäßes, wobei die Durchstoßeinheit der Ausgabeeinheit das Innengefäß durchstößt und so die zweite Ausgangssubstanz in das Vorratsgefäß eingebracht wird. Die Ausgabeeinheit wird dabei durch Drücken der Ausgabeeinheit in Richtung des Vorratsgefäßes oder durch Drehen der Schraubhülse in Richtung des Vorratsgefäßes bewegt. Um schneller ein einheitliches endgültiges Präparat zu erhalten, wird die Dosiervorrichtung vorzugsweise geschüttelt, um die erste und die zweite Ausgangssubstanz und gegebenenfalls die dritte oder weitere Ausgangssubstanzen zu vermischen.

Eine Verwendung der erfindungsgemäße Dosiervorrichtung ist die Applizierung von medizinischen Produkten, pharmazeutischen Produkten, kosmetischen Produkten, Reinigungsmitteln, Chemikalien, Lebensmitteln, Nahrungsergänzungsmitteln oder Flüssigwürzen, wobei auf die Verwendung von Konservierungsmitteln verzichtet werden kann. Im Falle von kosmetischen Produkten können beispielsweise Parfums ohne Alkohol und Konservierungsstoffe länger haltbar gemacht werden.

Weiterhin ist eine Verwendung der Dosiervorrichtung zur Applizierung von Präparaten, die Vitamine, Mineralstoffe, Enzyme, Co-Enzyme, Pflanzenextrakte, Bakterien, Hefen als einzelne Substanz oder Mischungen aus mehreren dieser Substanzen enthalten können, erfindungsgemäß.

Die vorliegende Erfindung wird anhand der nachfolgenden Figuren näher erläutert, ohne auf die in den Figuren dargestellten speziellen Ausführungen beschränkt zu sein.

Es zeigen
- Figuren 1A-E: einen Längsschnitt durch eine erfindungsgemäße Dosiervorrichtung mit Dosierpumpe in nicht aktivierter Position und in Gebrauchsposition;
- Figuren 2A-E: einen Längsschnitt durch eine weitere erfindungsgemäße Dosiervorrichtung mit Dosierpumpe in nicht aktivierter Position und in Gebrauchsposition;
- Figuren 3A-C: die Fertigungsreihenfolge einer erfindungsgemäßen Dosiervorrichtung;
- Figuren 4A und B: einen Längsschnitt durch eine als Einwegspritze ausgebildete erfindungsgemäße Dosiervorrichtung in nicht aktivierter Position; und
- Figur 5: einen Längsschnitt durch eine Dosierpumpe.

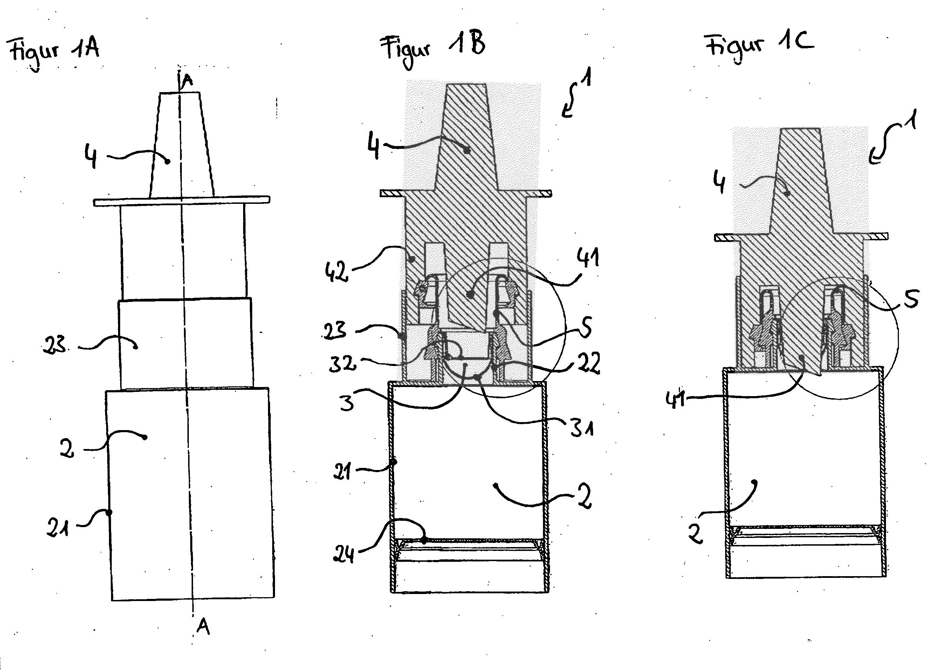

Figur 1A zeigt eine Frontalansicht einer erfinderischen Dosiervorrichtung 1 in nicht aktivierter Position, welche durch Drücken aktivierbar ist. In Figur 1B ist der in Figur 1A angedeutete Schnitt A-A durch die Dosiervorrichtung dargestellt.

Die Dosiervorrichtung 1 weist ein Vorratsgefäß 2, ein Innengefäß 3 und eine als Dosierpumpe 4 ausgebildete Ausgabeeinheit auf. Das Vorratsgefäß 2 enthält einen zylinderförmigen Gefäßrumpf 21, einen zylinderförmigen Vorratsgefäßhals 22, dessen Durchmesser geringer als der des Gefäßrumpfes 21 ist sowie einen Führungszylinder 23, dessen Durchmesser einen Wert zwischen dem des Gefäßbauches 21 und des Halses 22 annimmt. Außerdem weist das Vorratsgefäß 2 an seinem Boden einen Schleppkolben 24 auf. Das Innengefäß 3 enthält eine erste Schicht 31 und eine zweite Schicht 32. Die erste Schicht 31 ist mit ihrem Rand auf dem vom Gefäßrumpf 21 abgewandten Ende des Vorratsgefäßhalses 22 aufgeschweißt und hängt bauchig in den Hals 22 hinein. Die zweite Schicht 32 des Innengefäßes 3 ist ebenfalls mit ihrem Schichtrand auf dem vom Gefäßrumpf 21 abgewandten Ende des Halses 22 angeordnet und hängt leicht bauchig in den Hals 22 hinein.

Die Dosierpumpe 4 weist einen Einlassstutzen 41 sowie einen Hohlzylinder 42, welcher den Einlassstutzen 41 konzentrisch umläuft, auf. Im ihrem Inneren weist die Dosierpumpe 4 die folgenden, nicht dargestellten Komponenten auf: Pumpkolben, Pumpkammer und eine über einen Auslasskanal mit der Pumpkammer in Verbindung stehende Düse.

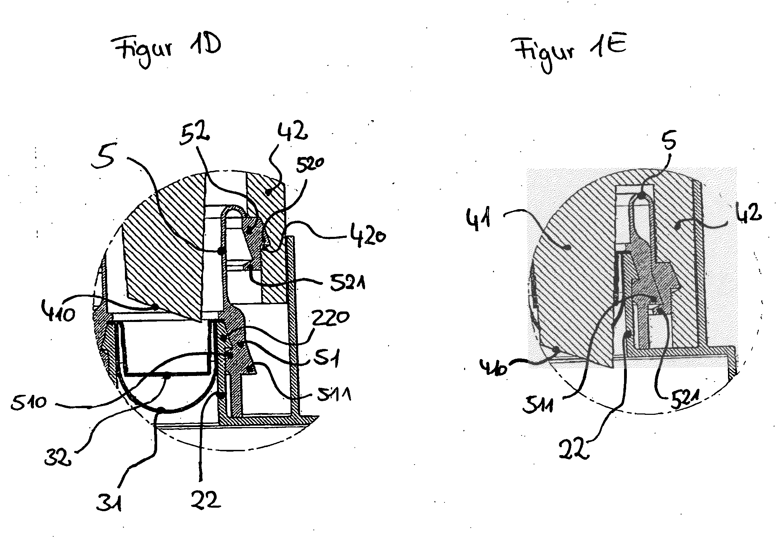

Eine dichtende Verbindung zwischen dem Vorratsgefäß 2 und der Dosierpumpe 4 wird durch einen flexiblen Kunststoffschlauch 5, welcher im Bereich des Halses 22 sowie des Hohlzylinders 42 befestigt ist, hergestellt. Der Kunststoffschlauch 5 ist in vergrößerter Ansicht in Figur 1D dargestellt. Figur 1D zeigt, dass der Hals 22 an seinem dem Gefäßrumpf 21 abgewandten Bereich eine Hinterschneidung 220 aufweist. Ähnlich weist auch der Zylinder 42 an seiner Innenseite eine Hinterschneidung 420 auf. Der Schlauch 5 weist in dem Bereich, mit welchem er über den Hals 22 des Vorratsgefäßes 2 gestülpt ist, eine Verdickung 51 auf, welche auf der Innenseite des Schlauches 5 eine Hinterschneidung 510 aufweist sowie auf der vom Hals 22 abgewandten Seite einen Vorsprung 511. Die Hinterschneidung 510 des Schlauches 5 und die Hinterschneidung 220 des Halses 20 greifen formschlüssig ineinander ein.

In dem Bereich 52, in welchem der Schlauch 5 mit dem Zylinder 42 in Kontakt ist, ist die Innenseite des Schlauches 5 nach außen gestülpt. Der Schlauch 5 weist dort auf der dem Zylinder 42 zugewandten Seite, d.h. auf der eigentlichen Innenseite des Schlauches 5, einen Vorsprung 520 auf, welcher mit der Hinterschneidung 420 des Hohlzylinders 42 in Eingriff ist. Desweiteren weist der Bereich 52 einen Vorsprung 521 auf, welcher an der vom Hohlzylinder 42 abgewandten Seite des Bereichs 52 angeordnet ist.

Um die in den Figuren 1A und B dargestellte erfindungsgemäße Dosiervorrichtung 1 in die Gebrauchsposition zu bringen, wird die Dosierpumpe 4 in Richtung des Vorratsgefäßes 2 bewegt. Dabei wird die erste 31 und die zweite 32 Schicht des Innengefäßes 3 von dem Einlassstutzen 41, welcher an seinem Ende angespitzt ist und einen Schneidekranz 410 aufweist, durchstoßen.

Figur 1C zeigt nun die Dosiervorrichtung 1 in Gebrauchsposition. Der Einlassstutzen 41 der Dosierpumpe 4 befindet sich nun im Bereich des Halses 22, in welchem zuvor das Innengefäß 3 positioniert war. Der Schlauch 5 ist in der Gebrauchsposition der Dosiervorrichtung 1 nach innen in den Pumpkopf, d.h. in den Bereich zwischen Hohlzylinder 42 und Einlassstutzen 41, eingerollt:

Figur 1E zeigt einen vergrößerten Ausschnitt eines Teils des Schlauches 5, welcher darstellt, dass der Vorsprung 511 im Bereich 51 des Schlauches 5 mit dem Vorsprung 521 des Bereichs 52 des Schlauches 5 in Eingriff ist und die Dosierpumpe 4 mit dem Vorratsgefäß 2 verrastet ist.

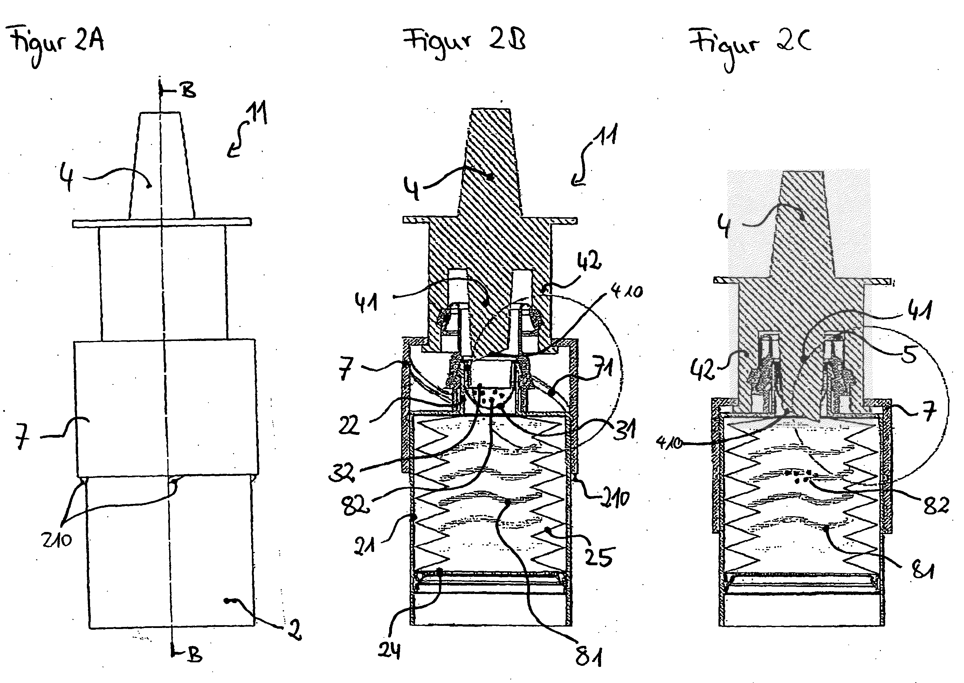

Figur 2A zeigt die Frontalansicht eines weiteren Ausführungsbeispiels einer erfindungsgemäßen Dosiervorrichtung 11, wobei die Aktivierung durch eine Schraubbewegung erfolgt. Figur 2B stellt einen in Figur 2A mit B-B bezeichneten Längsschnitt durch die Dosiervorrichtung dar.

Die Dosiervorrichtung weist wiederum ein Vorratsgefäß 2 mit einem Gefäßrumpf 21 und einem Vorratsgefäßhals 22 auf. In dem Vorratsgefäß 2 ist ein Faltenbalg 25 dargestellt, wobei der Faltenbalg 25 und der als Schleppkolbenboden 24 ausgebildete Boden einen Druckausgleich ermöglichen. Der Faltenbalg 25 ist mit einer flüssigen ersten Substanz 81 gefüllt.

Am abgewandten Ende des Halses 22 ist der Rand der ersten Schicht 31 und der zweiten Schicht 32 des Innengefäßes 3, welches mit einer pulverförmigen zweiten Substanz 82 gefüllt ist, abdichtend befestigt bzw. aufgeschweißt. Außerdem weist die Dosiervorrichtung 11 eine Dosierpumpe 4 mit einem Einlassstutzen 41, dessen dem Vorratsgefäß 2 zugewandtes Ende angespitzt ist und einen Schneidekranz 410 aufweist, und einem den Einlassstutzen 41 umlaufenden Hohlzylinder 42 auf. Die Dosierpumpe 4 ist wiederum über einen flexiblen Kunststoffschlauch 5, welcher wie der in der Beschreibung der Figuren 1A bis E beschriebene Schlauch 5 ausgebildet ist, mit dem Vorratsgefäßhals 22 verbunden.

Im Gegensatz zu der in den Figuren 1A bis E dargestellten Dosiervorrichtung 1 weist die Dosierpumpe 4 in Figur 2A in dem, dem Vorratsgefäß 2 zugewandten Bereich des Hohlzylinders 42 einen nach außen überstehenden Rand 421, d.h. einen vom Einlassstutzen 41 weg zeigenden Rand, auf. Des Weiteren weist die Dosiervorrichtung 11 eine Schraubhülse 7 zur Führung der Dosierpumpe 4 in Richtung des Vorratsgefäßes 2 auf. Die Schraubhülse 7 ist als Hohlzylinder ausgebildet, welcher an seinem der Dosierpumpe 4 zugewandten Ende einen nach innen überstehenden Rand 70 aufweist. Der Rand 70 greift in den Rand 421 ein. Des Weiteren weist die Schraubhülse an ihrer Innenseite Gewindegänge 71 auf, in welchen Stege 210, welche den Gefäßkörper 21 spiralförmig umlaufen, führbar sind.

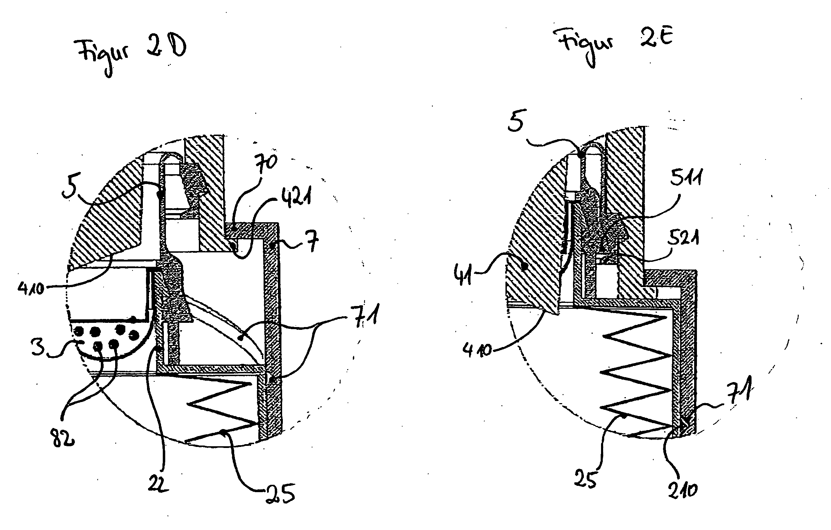

Figur 2D zeigt einen vergrößerten Ausschnitt des Schlauches 5. Zusätzlich sind die Schraubhülse 7 und ein Gewindegang 71 dargestellt.

Dreht nun ein Benutzer die Schraubhülse 7 im Uhrzeigersinn, so wird die Schraubhülse 7 nach unten geführt und gleichzeitig die Dosierpumpe 4 in Richtung des Vorratsgefäßes 2 gezogen. Dabei durchstößt der Einlassstutzen 41 das Innengefäß 3, so dass die Substanz aus dem Innengefäß 3 in das Vorratsgefäß 2 hineinfällt und mit der ersten Substanz vermischt wird. Figur 2C zeigt die Dosiervorrichtung 11 im aktivierten Zustand. Die Schraubhülse 7 ist bis zum Anschlag in Richtung des Uhrzeigersinns verdreht und die Dosierpumpe 4 ist so weit in Richtung des Gefäßrumpfes 21 bewegt, dass der Einlassstutzen 41 im Bereich des Halses 22 angeordnet ist. Wiederum ist der Schlauch 5 in den Zwischenraum zwischen Einlassstutzen 41 und Hohlzylinder 42 eingedreht. Wie in Figur 2E in vergrößerter Form dargestellt, sind außerdem der Vorsprung 511 und der Vorsprung 521 miteinander in Eingriff und die Dosierpumpe 4 ist somit auf dem Vorratsgefäß 2 aufgerastet.

In Figur 2E ist außerdem dargestellt, dass der Steg 210 des Gefäßrumpfes 21 im Gewindegang 71 der Schraubhülse 7 geführt ist.

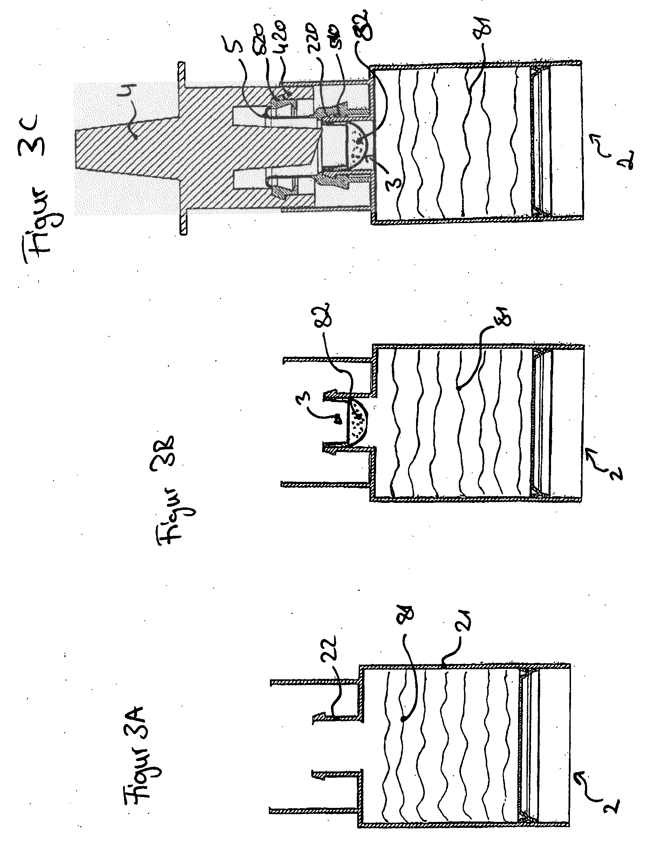

Die Figuren 3A bis C zeigen die Fertigungsreihenfolge einer erfindungsgemäßen Dosiervorrichtung 1. In einem ersten Schritt wird das Vorratsgefäß 21 mit einer flüssigen Substanz 81 befüllt, wobei zumindest der Bereich des Halses 22 leer bleibt. Das befüllte Vorratsgefäß 2 ist in Figur 3A dargestellt.

In einem zweiten Schritt wird ein bereits mit einem Pulver 82 befülltes Innengefäß 3 im Bereich des Halses 22 so angeordnet, dass das Vorratsgefäß 2 abgedichtet ist. Figur 3B zeigt das Vorratsgefäß 2 mit der Flüssigkeit 81 und das Innengefäß 3 mit dem Pulver 82, welches bauchförmig im Bereich des Halses 22 hängt.

In einem dritten Schritt wird nun die Dosierpumpe 4 und der flexible Kunststoffschlauch 5 auf das Vorratsgefäß 2 aufgesetzt. Dazu wird zunächst der Kunststoffschlauch mit der Hinterschneidung 520 in die Hinterschneidung 420 der Dosierpumpe 4 eingesetzt. Anschließend wird der Schlauch 5 über den Hals 22 gestülpt, so dass die Hinterschneidung 220 am Hals 22 mit der Hinterschneidung 510 des Schlauches 5 eingreift. Eine befüllte Dosiervorrichtung 1 ist in Figur 3C dargestellt.

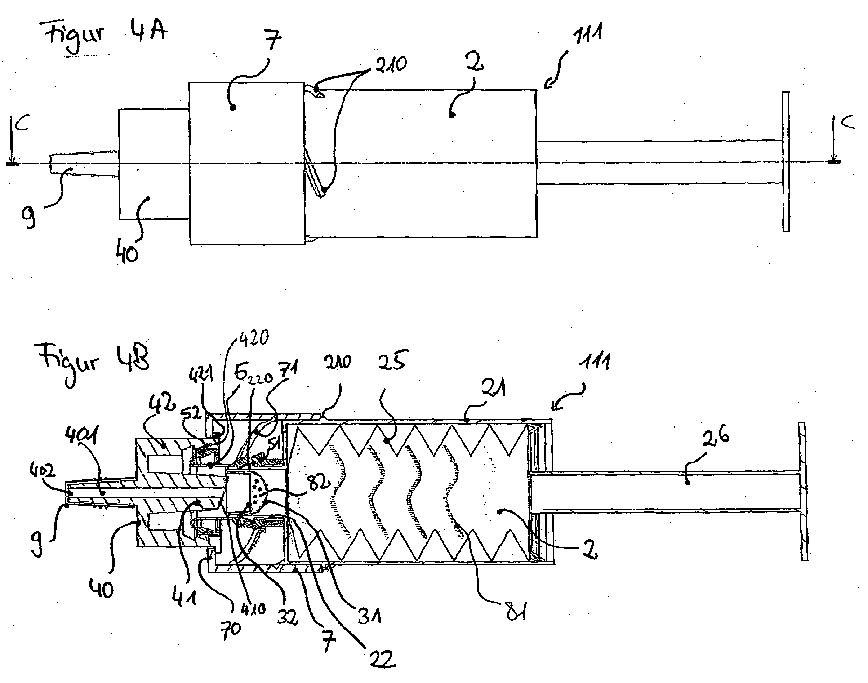

Wie in den Figuren 4A und B in Frontalansicht und als Längsschnitt C-C dargestellt ist, kann die erfindungsgemäße Dosiervorrichtung auch als Einwegspritze 111 ausgebildet sein.

Die Einwegspritze 111 weist ein Vorratsgefäß 2, ein Innengefäß 3, eine Ausgabekappe 40 und eine Schraubhülse 7 auf. Das Vorratsgefäß 2 enthält einen einem zylinderförmigen Gefäßrumpf 21, einem Gefäßhals 22 mit Hinterschneidungen 220 sowie einem Presskolben 26, welche zum Ausspritzen einer Flüssigkeit 81 in Richtung des Vorratsgefäßhalses 22 gedrückt wird. Die Flüssigkeit 81 befindet sich im Inneren eines Faltenbalgs 25, welche von dem Gefäßrumpf 21 umschlossen ist.

Das Innengefäß 3 besteht aus einer ersten 31 und einer zweiten 32 Schicht, zwischen welchen im nicht aktivierten Zustand der Spritze 111 ein Pulver 82 aufbewahrt ist. Die beiden Schichten 31 und 32, welche rund ausgebildet sind, sind mit ihrem Rand auf das dem Vorratsgefäß 2 abgewandten Ende des Vorratsgefäßhalses 22 abdichten aufgebracht bzw. aufgeklebt.

Die Ausgabekappe 40 weist einen Einlassstutzen 41 mit einem schrägen Schneidekranz 410 und einen im Einlassstutzen 41 verlaufenden Kanal 401, durch welchen das endgültige flüssige Präparat durch eine Auslassöffnung 402 in der Gebrauchsposition austreten kann. Die Auslassöffnung 402 ist mit einer Verschlusskappe 9 abgedeckt und abgedichtet.

Die Ausgabekappe 40 weist weiterhin einen Hohlzylinder 42 auf, welcher den Einlassstutzen 41 umgibt. Der Hohlzylinder 42 enthält an seiner Innenseite Hinterschneidungen 420 und an seinem dem Vorratsgefäß 2 zugewandten Ende einen nach außen überstehenden Rand 421.

Die Ausgabekappe 40 ist wie in den in den Figuren 1A bis E und 2A bis E darstellten Ausführungsbeispielen beschrieben über einen flexiblen Kunststoffschlauch 5, wie er aus den vorherigen Beispielen bereits bekannt ist, mit dem Vorratsgefäß 2 luft- und wasserdicht verbunden. Der Schlauch 5 ist mit seinem Bereich 51 über den Vorratsgefäßhals 22 gestülpt und mit seinem Bereich 52 mit dem Hohlzylinder 42 des Ausgabekappe 40 in Eingriff. In der Gebrauchsposition sind die Bereiche 51 und 52 des Schlauches 5 verrastet.

Die Schraubhülse 7 ist wie im Beispiel der Dosiervorrichtung 11 der Figuren 2A bis E ausgebildet. Sie weist an der Innenseite ihres zylindrischen Körpers Gewindegänge auf, in welchen die Stege 210 an der Außenseite des Vorratsgefäßrumpfes 21 führbar sind. Mit ihrem Rand 70 ist die Schraubhülse 7 mit dem Rand 421 der Ausgabekappe 40 in Eingriff.

Zur Aktivierung wird die Ausgabekappe 40 mit dem Einlassstutzen 41 durch eine Viertelumdrehung der Schraubhülse 7 in Richtung des Vorratsgefäßrumpfes 21 gezogen und das Innengefäß 3 vom Einlassstutzen durchstoßen, so dass das Pulver 82 in die flüssige Substanz 81 eingebracht wird. In einer alternativen Variante kann die Aktivierung auch durch Drücken erfolgen. Um den Mischungs- bzw. Lösungsvorgang zu beschleunigen kann die Einwegspritze geschüttelt werden.

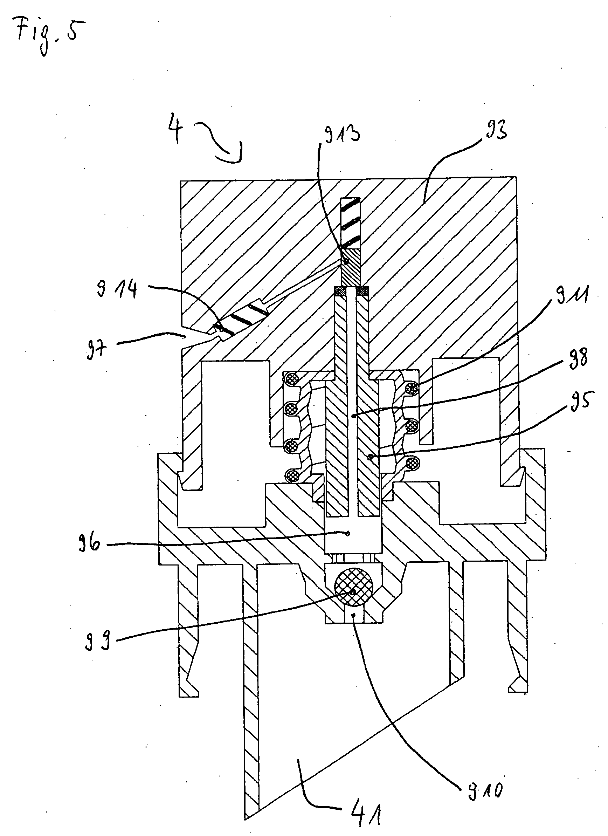

In Figur 5 ist ein Längsschnitt durch eine für die erfindungsgemäße Dosiervorrichtung 1 einsetzbare Dosierpumpe 4 dargestellt. Das flüssige Präparat gelangt nach Betätigung des Betätigungskörpers 93 durch einen Einlassstutzen 41, der die Einlassöffnung 910 mit dem Einlassventil 99 umgibt, und durch die Pumpkammer 96 sowie die Zuleitung 98, die von dem Pumpkolben 95 umgeben ist, in das Auslassventil 913. Bevor die Flüssigkeit durch die Düse 97 ausgestoßen wird, passiert sie die Silberspirale 914. Durch die Silberspirale 914 ist eine effiziente Sterilhaltung der Flüssigkeit im Bereich der Düse 97 gewährleistet. Die Rückstellfedern 911 dienen der selbsttätigen Rückführung des Betätigungskörpers 93 in die Ausgangsposition.

Die vorliegende Erfindung ermöglicht eine langfristige Lagerung von Präparaten, da nicht nur eine hohe Dichtigkeit der Dosiervorrichtung gegeben ist, sondern eine Trennung der Substanzen während einer Lagerungszeit realisiert ist, wodurch die Haltbarkeit der einzelnen Substanzen erhöht wird.

## Patentansprüche

1. Dosiervorrichtung (1,11,111) zur dosierten Ausgabe eines flüssigen Präparates aus zumindest einer ersten (81) und einer zweiten (82) Ausgangssubstanz, wobei die Dosiervorrichtung (1,11,111) ein Vorratsgefäß (2) zur Aufnahme zumindest der ersten Ausgangssubstanz (81) mit zumindest einer Öffnung und eine Ausgabeeinheit mit zumindest einem Durchlasskanal mit zwei Kanalöffnungen aufweist, wobei die Ausgabeeinheit (4,40) und das Vorratsgefäß (2) so zueinander angeordnet sind, dass eine der Kanalöffnungen und die Öffnung des Vorratsgefäßes (2) einander zugewandt sind und die Ausgabeeinheit (4,40) im Bereich der Öffnung des Vorratsgefäßes (2) dichtend mit dem Vorratsgefäß (2) verbunden ist,
wobei der dem Vorratsgefäß (2) zugewandte Bereich der Ausgabeeinheit (4,40) als Durchstoßeinheit ausgebildet ist, welche in die Öffnung des Vorratsgefäßes (2) einführbar und/oder in dieser führbar ist; und
wobei im Bereich der Öffnung des Vorratsgefäßes zwischen Vorratsgefäß (2) und Ausgabeeinheit (4,40) ein zumindest gegenüber dem Vorratsgefäß (2) abgedichtetes Innengefäß (3) zur Aufnahme der zweiten Ausgangssubstanz (82) angeordnet ist, wobei das Innengefäß (3) mit Hilfe der Durchstoßeinheit der Ausgabeeinheit (4,40) durchstoßbar ist,
**dadurch gekennzeichnet, dass**
die Ausgabeeinheit (4,40) einen Hohlzylinder (42) oder ein Hohlprisma aufweist, welche die Durchstoßeinheit umgeben; dass
der Hohlzylinder (42) oder das Hohlprisma an ihrer Innenseite zumindest bereichsweise Hinterschneidungen (420) aufweisen; dass
das Vorratsgefäß (2) im Bereich seiner Öffnung Hinterschneidungen (220) aufweist, wobei nach dem Durchstoßen des Innengefäßes (2) die Hinterschneidungen des Hohlzylinders (42) oder des Hohlprismas und die des Vorratsgefäßes in direktem oder indirektem Eingriff sind; und dass
die Ausgabeeinheit (4,40) und die Öffnung des Vorratsgefäßes (2) mittels eines flexiblen Kunststoffschlauches (5) verbunden sind, wobei der Kunststoffschlauch (5) bevorzugt in den Bereichen, welche mit dem Hohlzylinder (42) oder Hohlprisma und/oder dem Vorratsgefäß (2) in Verbindung sind, zumindest jeweils mindestens eine Verdickung aufweist, die mit den Hinterschneidungen (420) auf der Innenseite des Hohlzylinders oder des Hohlprismas und/oder den Hinterschneidungen (220) im Bereich der Öffnung des Vorratsgefäßes (2) im Eingriff ist.

2. Dosiervorrichtung (1,11,111) nach dem vorhergehenden Anspruch, **dadurch gekennzeichnet, dass** die Ausgabeeinheit (4,40) nach dem Durchstoßen des Innengefäßes (3) mit dem Vorratsgefäß (2) verrastet ist.

3. Dosiervorrichtung (1,11,111) nach einem der vorhergehenden Ansprüche, **dadurch gekennzeichnet, dass** die Durchstoßeinheit der Ausgabeeinheit (4,40) ein angespitzter Einlassstutzen oder ein Schneidekranz (410), ein Sägekranz, ein Zackenkranz, insbesondere ein schräger Schneide-, Säge- oder Zackenkranz, mindestens eine Zinke oder mindestens ein Dorn ist.

4. Dosiervorrichtung (1,11,111) nach einem der vorhergehenden Ansprüche, **dadurch gekennzeichnet, dass** die Ausgabeeinheit (4,40) eine Dosierpumpe ist, welche einen Einlasskanal, einen Pumpkolben (95), eine Pumpkammer (96) und eine über einen Auslasskanal mit der Pumpkammer verbundene Düse (97) aufweist.

5. Dosiervorrichtung (1,11,111) nach einem der Ansprüche 1 bis 3, **dadurch gekennzeichnet, dass** die Dosiervorrichtung eine Einwegspritze (111) ist, welche an der der Ausgabeeinheit (40) abgewandten Vorratsgefäßseite (2) einen Presskolben (26) aufweist.

6. Dosiervorrichtung (1,11,111) nach einem der vorhergehenden Ansprüche, **dadurch gekennzeichnet, dass** das Vorratsgefäß (2) eine Druckausgleichsvorrichtung enthält.

7. Dosiervorrichtung (1,11,111) nach einem der vorhergehenden Ansprüche, **dadurch gekennzeichnet, dass** das Innengefäß (3) vor dem Durchstoßen des Innengefäßes als geschlossenes Gefäß ausgebildet ist und/oder das Vorratsgefäß (2) dichtend verschließt.

8. Dosiervorrichtung (1,11,111) nach einem der vorhergehenden Ansprüche, **dadurch gekennzeichnet, dass** das Innengefäß (3) aus einem Metall, insbesondere Aluminium, aus einem Kunststoff, insbesondere Poly-chlortrifluorethylen, Cyclo-Olefin-Polymer, Polypropylen, Polyethylen, Polymerfolien, Aluminium mit Kunststoffbeschichtung oder aus Glas besteht oder eines dieser Materialien enthält.

9. Dosiervorrichtung (1,11,111) nach einem der vorhergehenden Ansprüche, **dadurch gekennzeichnet, dass** das Vorratsgefäß (2) einen Hohlzylinder (42) oder ein Hohlprisma aufweist, wobei der Hohlzylinder (42) oder das Hohlprisma die Öffnung des Vorratsgefäßes umgebe, und der Hohlzylinder (42) oder das Hohlprisma bevorzugt als Führung für die Ausgabeeinheit (4,40) dient, um ein Verkanten zu vermeiden.

10. Dosiervorrichtung (1,11,111) nach einem der vorhergehenden Ansprüche, **dadurch gekennzeichnet, dass** die Dosiervorrichtung eine Schraubhülse (7) zur Führung der Ausgabeeineheit (4,40) in Richtung des Vorratsgefäßes aufweist, wobei die Schraubhülse (7) vorzugsweise ein oder mehrere Gewindegänge (71) aufweist.

11. Dosiervorrichtung (1,11,111) nach einem der vorhergehenden Ansprüche, **dadurch gekennzeichnet, dass** das Innengefäß (3) und/oder das Vorratsgefäß (2) Schutzgas und/oder Inertgas enthalten, um den Inhalt vor Oxidation und/oder Hydrolyse zu schützen und/oder die erste Ausgangssubstanz (81) mit Schutzgas und/oder Inertgas, gegebenenfalls vor dem Befüllen, durchsetzt ist.

12. Dosiervorrichtung (1,11,111) nach einem der vorhergehenden Ansprüche, **dadurch gekennzeichnet dass** das Innengefäß (3) und/oder das Vorratsgefäß (2) unter einer Atmosphäre aus Schutzgas und/oder Inertgas befüllt ist und das Schutzgas bevorzugt ausgewählt ist aus Stickstoff, Kohlenstoffdioxid oder Mischungen hiervon und das Inertgas bevorzugt ausgewählt ist aus Stickstoff, Edelgasen, wie z.B. Helium oder Argon, oder Mischungen hiervon.

13. Dosiervorrichtung (1,11,111) nach einem der vorhergehenden Ansprüche, **dadurch gekennzeichnet, dass** im Vorratsgefäß (2) ein Faltenbalg (25) angeordnet ist, wodurch eine hermetische Abdichtung der Dosiervorrichtung bis zum Ende der Verwendung gewährleistet ist.

14. Verfahren zum Betreiben der Dosiervorrichtung (1,11,111) nach einem der vorhergehenden Ansprüche, wobei
die Ausgabeeinheit (4,40) in Richtung des Vorratsgefäßes (2) bewegt wird und die Durchstoßeinheit das Innengefäß (3) durchstößt.

15. Verfahren nach dem vorhergehenden Anspruch, **dadurch gekennzeichnet, dass** die Dosiervorrichtung (1,11,111) nach dem Durchstoßen des Innengefäßes (3) geschüttelt wird.

16. Verwendung der Dosiervorrichtung (1,11,111) nach einem der Ansprüche 1 bis 13 zur Applizierung von medizinischen Produkten, pharmazeutischen Produkten, kosmetischen Produkten, Reinigungsmitteln, Chemikalien, Lebensmitteln, Nahrungsergänzungsmitteln oder Flüssigwürzen, wobei auf die Verwendung von Konservierungsmitteln verzichtet werden kann, sowie zur Applizierung von Präparaten, die Vitamine, Mineralstoffe, Enzyme, Co-Enzyme, Pflanzenextrakte, Bakterien, Hefen als einzelne Substanz oder Mischungen aus mehreren dieser Substanzen enthalten können.

## Claims

1. Metering device (1,11,111) for metered output of a liquid preparation comprising at least a first (81) and a second (82) starting substance, the metering device (1,11,111) having a storage vessel (2), for receiving at least the first starting substance (81), with at least one opening, and an output unit having at least one passage channel with two channel openings, the output unit (4,40) and the storage vessel (2) being disposed relative to each other such that one of the channel openings and the opening of the storage vessel (2) are orientated towards each other and the output unit (4,40), in the region of the opening of the storage vessel (2), is connected to the storage vessel (2) in a sealed manner,
the region of the output unit (4,40) orientated towards the storage vessel (2) being configured as a penetration unit which can be introduced into the opening of the storage vessel (2) and/or can be guided in the latter; and in the region of the opening of the storage vessel between storage vessel (2) and output unit (4,40), an inner vessel (3), which is sealed at least relative to the storage vessel (2), for receiving the second starting substance (82) being disposed, the inner vessel (3) being able to be penetrated with the help of the penetration unit of the output unit (4,40),
**characterised in that**
the output unit (4,40) has a hollow cylinder (42) or a hollow prism which surround the penetration unit; **in that**
the hollow cylinder (42) or the hollow prism have undercuts (420) on their inside at least in regions; **in that**
the storage vessel (2) has undercuts (220) in the region of its opening, the undercuts of the hollow cylinder (42) or of the hollow prism and those of the storage vessel preferably being in direct or indirect engagement after penetration of the inner vessel (2); and **in that**
the output unit (4,40) and the opening of the storage vessel (2) are connected by means of a flexible plastic material tube (5), the plastic material tube (5) having at least respectively at least one thickened portion preferably in the regions which are connected to the hollow cylinder (42) or hollow prism and/or to the storage vessel (2), which thickened portion is in engagement with the undercuts (420) on the inside of the hollow cylinder or of the hollow prism and/or with the undercuts (220) in the region of the opening of the storage vessel (2).

2. Metering device (1,11,111) according to the preceding claim, **characterised in that** the output unit (4,40), after penetration of the inner vessel (3), is locked to the storage vessel (2).

3. Metering device (1,11,111) according to one of the preceding claims, **characterised in that** the penetration unit of the output unit (4,40) is a pointed inlet connection piece or a cutting collar (410), a saw collar, a toothed collar, in particular a diagonal cutting-, saw- or toothed collar, at least one tine or at least one mandril.

4. Metering device (1,11,111) according to one of the preceding claims, **characterised in that** the output unit (4,40) is a metering pump which has an inlet channel, a pump piston (95), a pump chamber (96) and a nozzle (97) which is connected to the pump chamber via an outlet channel.

5. Metering device (1,11,111) according to one of the preceding claims 1 to 3, **characterised in that** the metering device is a disposable syringe (111) which has a plunger (26) on the side of the storage vessel (2) which is orientated away from the output unit (40).

6. Metering device (1,11,111) according to one of the preceding claims, **characterised in that** the storage vessel (2) contains a pressure-equalisation device.

7. Metering device (1,11,111) according to one of the preceding claims, **characterised in that** the inner vessel (3), before penetration of the inner vessel, is configured as a closed vessel and/or the storage vessel (2) is closed in a sealed manner.

8. Metering device (1,11,111) according to one of the preceding claims, **characterised in that** the inner vessel (3) consists of a metal, in particular aluminium, of a plastic material, in particular polychlorotrifluoroethylene, cycloolefin polymer, polypropylene, polyethylene, polymer films, aluminium with a plastic material coating or of glass or comprises one of these materials.

9. Metering device (1,11,111) according to one of the preceding claims, **characterised in that** the storage vessel (2) has a hollow cylinder (42) or a hollow prism, the hollow cylinder (42) or the hollow prism surrounding the opening of the storage vessel, and the hollow cylinder (42) or the hollow prism preferably serving as a guide for the output unit (4,40) in order to avoid tilting.

10. Metering device (1,11,111) according to one of the preceding claims, **characterised in that** the metering device has a screwed bush (7) for guiding the output unit (4,40) in the direction of the storage vessel, the screwed bush (7) preferably having one or more threads (71).

11. Metering device (1,11,111) according to one of the preceding claims, **characterised in that** the inner vessel (3) and/or the storage vessel (2) contain protective gas and/or inert gas in order to protect the contents from oxidation and/or hydrolysis and/or protective gas and/or inert gas is passed through the first starting substance (81), if necessary before filling.

12. Metering device (1,11,111) according to one of the preceding claims, **characterised in that** the inner vessel (3) and/or the storage vessel (2) is filled under an atmosphere comprising protective gas and/or inert gas and the protective gas is preferably selected from nitrogen, carbon dioxide or mixtures hereof and the inert gas is preferably selected from nitrogen, noble gases, such as e.g. helium or argon, or mixtures hereof.

13. Metering device (1,11,111) according to one of the preceding claims, **characterised in that** bellows (25) are disposed in the storage vessel (2), as a result of which a hermetic seal of the metering device until the end of use is ensured.

14. Method for operating the metering device (1,11,111) according to one of the preceding claims,
the output unit (4,40) being moved in the direction of the storage vessel (2) and the penetration unit penetrating the inner vessel (3).

15. Method according to the preceding claim, **characterised in that** the metering device (1,11,111) is shaken after penetration of the inner vessel (3).

16. Use of the metering device (1,11,111) according to one of the claims 1 to 13 for administering medicinal products, pharmaceutical products, cosmetic products, cleaning agents, chemicals, foodstuffs, food supplements or liquid seasonings, the use of preservatives being able to be dispensed with, and for administering preparations which can contain vitamins, minerals, enzymes, coenzymes, plant extracts, bacteria, yeasts as individual substance or mixtures of a plurality of these substances.

## Revendications

1. Dispositif de dosage (1, 11, 111) pour la distribution dosée d'une préparation liquide composée d'au moins une première substance de départ (81) et d'une deuxième (82) substance de départ, le dispositif de dosage (1, 11, 111) présentant un récipient de stockage (2) pour la réception d'au moins la première substance de départ (81) avec au moins une ouverture et une unité de distribution avec au moins un canal de passage avec deux ouvertures de canal, l'unité de distribution (4, 40) et le récipient de stockage (2) étant disposés l'un par rapport à l'autre de telle sorte que l'une des ouvertures de canal et l'ouverture du récipient de stockage (2) sont tournées l'une vers l'autre, et l'unité de distribution (4, 40) étant, dans la zone de l'ouverture du récipient de stockage (2), raccordée de façon étanche au récipient de stockage (2),
la zone de l'unité de distribution (4, 40) tournée vers le récipient de stockage (2) étant constituée en tant qu'unité de perçage qui peut être introduite dans l'ouverture du récipient de stockage (2) et/ou peut être guidée dans celle-ci ; et ;
un récipient intérieur (3) rendu étanche au moins vis-à-vis du récipient de stockage (2) étant disposé dans la zone de l'ouverture du récipient de stockage entre le récipient de stockage (2) et l'unité de distribution (4, 40) pour la réception de la deuxième substance de départ (82), le récipient intérieur (3) pouvant être percé à l'aide de l'unité de perçage de l'unité de distribution (4, 40),
**caractérisé en ce que**
l'unité de distribution (4, 40) présente un cylindre creux (42) ou un prisme creux qui entourent l'unité de percement ; **en ce que**
le cylindre creux (42) ou le prisme creux présentent sur leur côté intérieur au moins par tronçons des contre-dépouilles (420) ; **en ce que**
le récipient de stockage (2) présente des contre-dépouilles (220) dans la zone de son ouverture, les contre-dépouilles du cylindre creux (42) ou du prisme creux et celles du récipient de stockage sont en prise directe ou indirecte après le perçage du récipient intérieur (2); et **en ce que**
l'unité de distribution (4, 40) et l'ouverture du récipient de stockage (2) sont raccordées par le bilais d'un tuyau en matière plastique (5) flexible, le tuyau en matière plastique (5) présentant, de préférence dans les zones qui sont en liaison avec le cylindre creux (42) ou le prisme creux et/ou le récipient de stockage (2), au moins chacune au moins un épaississement qui est en prise avec les contre-dépouilles (420) sur le côté intérieur du cylindre creux ou du prisme creux et/ou avec les contre-dépouilles (220) dans la zone de l'ouverture du récipient de stockage (2).

2. Dispositif de dosage (1, 11, 111) selon la revendication précédente, **caractérisé en ce que** l'unité de distribution (4, 40) est encliquetée avec le récipient de stockage (2) après le perçage du récipient intérieur (3).

3. Dispositif de dosage (1, 11, 111) selon l'une des revendications précédentes, **caractérisé en ce que** l'unité de perçage de l'unité de distribution (4, 40) est une tubulure d'entrée taillée en pointe ou un disque de coupe (410), un disque de sciage, un disque de dentelage, en particulier un disque de coupe (410), un disque de sciage ou un disque de dentelage oblique, au moins une dent ou au moins un mandrin.

4. Dispositif de dosage (1, 11, 111) selon l'une des revendications précédentes, **caractérisé en ce que** l'unité de distribution (4, 40) est une pompe de dosage qui présente un canal d'entrée, un piston de pompe (95), une chambre de pompage (96) et une buse (97) raccordée à la chambre de pompage par le biais d'un canal de sortie.

5. Dispositif de dosage (1, 11, 111) selon l'une des revendications 1 à 3, **caractérisé en ce que** le dispositif de dosage est une seringue à usage unique (111) qui présente un piston de compression (26) sur le côté du récipient de stockage (2) qui est éloigné de l'unité de distribution (40).

6. Dispositif de dosage (1, 11, 111) selon l'une des revendications précédentes, **caractérisé en ce que** le récipient de stockage (2) contient un dispositif d'équilibrage de pression.

7. Dispositif de dosage (1, 11, 111) selon l'une des revendications précédentes, **caractérisé en ce que**, avant le perçage du récipient intérieur, le récipient intérieur (3) est constitué en tant que récipient fermé et/ou **en ce que** le récipient de stockage (2) ferme de façon étanche.

8. Dispositif de dosage (1, 11, 111) selon l'une des revendications précédentes, **caractérisé en ce que** le récipient intérieur (3) est réalisé en métal, en particulier en aluminium, en matière plastique, en particulier polychlorotrifluoréthylène, polymère cyclo-oléfine, polypropylène, polyéthylène, films polymères, en aluminium revêtu de matière plastique, ou en verre ou un de ces matériaux.

9. Dispositif de dosage (1, 11, 111) selon l'une des revendications précédentes, **caractérisé en ce que** le récipient de stockage (2) présente un cylindre creux (42) ou un prisme creux, le cylindre creux (42) ou le prisme creux entourant l'ouverture du récipient de stockage, et le cylindre creux (42) ou le prisme creux servant de préférence de guidage pour l'unité de distribution (4, 40) afin d'éviter un gauchissement.

10. Dispositif de dosage (1, 11, 111) selon l'une des revendications précédentes, **caractérisé en ce que** le dispositif de dosage présente une douille filetée (7) pour le guidage de l'unité de distribution (4, 40) en direction du récipient de stockage, la douille filetée (7) présentant de préférence un ou plusieurs pas de filetage (71).

11. Dispositif de dosage (1, 11, 111) selon l'une des revendications précédentes, **caractérisé en ce que** le récipient intérieur (3) et/ou le récipient de stockage (2) contiennent du gaz de protection et/ou du gaz inerte afin de protéger le contenu de l'oxydation et/ou de l'hydrolyse et/ou **en ce que** la première substance de départ (81) est chargée de gaz de protection et/ou de gaz inerte, éventuellement avant le remplissage.

12. Dispositif de dosage (1, 11, 111) selon l'une des revendications précédentes, **caractérisé en ce que** le récipient intérieur (3) et/ou le récipient de stockage (2) est rempli dans une atmosphère de gaz de protection et/ou de gaz inerte, et **en ce que** le gaz de protection est de préférence sélectionné parmi l'azote, le dioxyde de carbone ou des mélanges de ceux-ci, et **en ce que** le gaz inerte est de préférence sélectionné parmi l'azote, des gaz rares, comme par exemple l'hélium ou l'argon, ou des mélanges de ceux-ci.

13. Dispositif de dosage (1, 11, 111) selon l'une des revendications précédentes, **caractérisé en ce qu'**un soufflet (25) est disposé dans le récipient de stockage (2), ce qui garantit une étanchéité hermétique du dispositif de dosage jusqu'à la fin de l'utilisation.

14. Procédé de fonctionnement du dispositif de dosage (1, 11, 111) selon l'une des revendications précédentes,
l'unité de distribution (4, 40) étant déplacée en direction du récipient de stockage (2) et l'unité de perçage perçant le récipient intérieur (3).

15. Procédé selon la revendication précédente, **caractérisé en ce que** le dispositif de dosage (1, 11, 111) est secoué après le perçage du récipient intérieur (3).

16. Utilisation du dispositif de dosage (1, 11, 111) selon l'une des revendications 1 à 13 pour l'application de produits médicaux, de produits pharmaceutiques, de produits cosmétiques, de détergents, de produits chimiques, de produits alimentaires, de compléments alimentaires ou de condiments liquides, la renonciation à l'utilisation de conservateurs étant possible, ainsi que pour l'application de préparations qui peuvent contenir des vitamines, des matières minérales, des enzymes, de co-enzymes, des extraits de plantes, des bactéries, des levures en tant que substances individuelle ou des mélanges de plusieurs de ces substances.
